# EUROPEAN PATENT SPECIFICATION

(11) **EP 1 601 632 B1**
(45) Date of publication and mention of the grant of the patent: **25.08.2010**
(21) Application number: 04704275.9
(22) Date of filing: 22.01.2004
(51) Int. Cl.: A61L 27/56

(54) **BONE SUBSTITUTE MATERIAL**
KNOCHENERSATZMATERIAL
MATERIAU DE SUBSTITUTION OSSEUSE

(30) Priority: 23.01.2003 GB 0301573; 12.08.2003 GB 0318902
(43) Date of publication of application: 07.12.2005
(73) Proprietor: UNIVERSITY OF BATH, Claverton Down, Bath, BA2 7AY (GB)
(72) Inventor: TURNER, Irene Gladys, Bradford on Avon, Wiltshire BA15 1QG (GB); MILES, Anthony William, Bath BA2 3JZ (GB); CASEY, Brian Paul, Fairfield Park, Bath BA1 6JH (GB); HSU, Yu-Hsiu, Bath BA1 2BN (GB)
(74) Representative: Bardo, Julian Eason
(86) International application number: PCT/GB2004/000253
(87) International publication number: WO 2004/065329

(56) References cited:
- WO-A-00/20353
- WO-A-01/94274
- WO-A-02/11781
- US-A- 6 136 029
- MILOSEVSKI M ET AL: "Preparation and properties of dense and porous calcium phosphate" CERAMICS INTERNATIONAL, ELSEVIER APPLIED SCIENCE PUBL, BARKING, ESSEX, GB, vol. 25, no. 8, December 1999 (1999-12), pages 693-696, XP004362781 ISSN: 0272-8842
- TANCRED D C ET AL: "A synthetic bone implant macroscopically identical to cancellous bone" BIOMATERIALS, ELSEVIER SCIENCE PUBLISHERS BV., BARKING, GB, vol. 19, no. 24, December 1998 (1998-12), pages 2303-2311, XP004168865 ISSN: 0142-9612

## Description

This invention relates to a method of fabricating a bone substitute material and to a bone substitute material that can be produced by such a method.

An important parameter of a bone substitute material is its porosity. In the past, two methods have commonly been used to fabricate such porous materials. A first method involves embedding organic particles in a ceramic body and subsequently burning out the organic particles to leave pores in the implant material. A second method involves the use of a porous substrate into which a ceramic slip is introduced. After drying the slip, heating serves both to burn out the substrate material and to sinter the slip. An example of such a method is described in a paper entitled "Hydroxyapatite-based porous aggregates: physico-chemical nature, structure, texture and architecture" by M. Fabbri, G. C. Celotti and A. Ravaglioli (Biomaterials 1995, Vol 16, No. 3, p 225). This invention relates to an improved method of the second type involving the use of a porous substrate.

In the method described in the paper referred to above, a ceramic slip was introduced into a spongy body taking care to leave its pores open. The body was then first dried and then sintered producing final products with a porosity in the range of 70 to 85% with densities in the region of 0.5 to 0.9g/cm³. Such a product clearly has a high porosity but the mechanical strength of the product, described by the authors as satisfactory when allowing for use under loads that are not too high, is clearly not sufficient for many applications.

WO 01/94274 describes a method for producing a ceramic material, which may be used as a bone substitute material, by introducing a ceramic slip into a foam material, burning out the foam material and sintering the ceramic slip to form the ceramic material. The ceramic slip introduced into the foam material coats the cell walls of the material and excess slip is removed.

US 6,136,029 describes various bone substitute materials and methods for making them. In one example, a ceramic slip is introduced into a reticulated foam to coat the struts (cell walls) of the foam. The reticulated foam may be immersed in the slip and then removed and drained to remove excess slip. The coated foam is then heated first to burn away the foam and then to sinter the product. The sintered product is said to form a rigid, light framework structure that mimics the configuration of the reticular struts.

In a paper entitled "Prepration and properties of dense and porous calcium phosphate" in Ceramics International 25 (1999) 693-696, procedures for coating bone replacements by coating the struts of polyurethane foam with a ceramic slip are described. After coating, the resultant structure is fired to pyrolyze the foam and to sinter the ceramic material.

WO 00/20353 and WO 02/11781 describe methods of forming bone substitute materials by foaming ceramic materials to generate porous materials. In a paper entitled "A synthetic bone implant macroscopically identical to cancellous bone" in Biomaterials 19 (1998) 2303-2311, a process for making a bone substitute material by introducing wax into cancellous bone and then introducing a ceramic slip into the wax is described and in that way a substitute material that is a positive replica of the original bone material is obtained.

It is an object of the invention to provide an improved method of fabricating a bone substitute material and to provide an improved material that can be produced by such a method.

According to the invention there is provided a method of fabricating a bone substitute material, the method comprising the steps of:
providing a foam material having an open cell structure,
introducing a ceramic slip into the cells of the foam material,
removing the foam material,
sintering the ceramic slip to form a bone substitute material,
**characterized in that:**
when the ceramic slip is introduced into the cells of the foam material, the cells of the foam material are substantially filled, and
the bone substitute material formed by the method is substantially a negative image of the foam material.

We have found that by adopting the method defined above it is possible, by suitable selection of the foam material, to obtain a bone substitute material of a chosen pore size and a chosen and relatively high degree of interconnection between the pores. In the bone substitute material produced by this method, the pores in the material (other than the micropores of less than 10 µm equivalent diameter) are generally in fluid communication with all adjacent pores; also walls around the pores are generally connected to adjacent walls extending in other directions. Also, it is possible to obtain a material that has consistent properties throughout its volume, or if desired and as explained below, a material that has a chosen variation in properties from one region to another.

An important feature of the method is that the cells of the foam material are substantially filled with the ceramic slip rather than the cell walls alone being coated as in the method described in the paper referred to above. In the latter case, the resulting product would be a positive image of the foam material but the ceramic parts of the structure would be hollow and therefore less strong.

The foam material may be removed by any suitable process, including for example immersion of the filled foam material in a solvent. It is preferred, however, that the step of removing the foam material comprises heating the material. The foam material may be a polymeric foam material, for example a polyurethane foam material, and may be removed by combustion.

When the foam material is removed by heating, the method preferably comprises a first heating step in which the foam material is removed and a second, subsequent, heating step in which the ceramic slip is heated to a higher temperature and is sintered. Although the two heating steps may be merged into a single step, it is preferred that the first heating step is a gentle step allowing the foam material to be removed gradually. Preferably the temperature to which the filled foam material is heated does not exceed 800°C. Also, the temperature of the filled foam material is preferably increased slowly, preferably at a rate of less than 200°C/hr and more preferably less than 100°C/hr. In an example of the invention described below, the filled foam material is raised to a temperature of 600°C at a rate of 60°C/hr in the first heating step; the material is then maintained at a temperature of 600°C for 1 hour.

The heating step for sintering, which is the second heating step in the case where there are first and second heating steps, preferably involves heating the filled material to over 1,000°C. Preferably the material is maintained at a temperature of over 1,000°C for more than 1 hour. In the example of the invention described below, the second heating step comprises raising the temperature from 600°C to 1280°C at a rate of 120°C/hr and then maintaining the material at 1280°C for 4 hours. Subsequently the material is cooled back to room temperature at the rate of 200°C/hr.

In order to promote substantial filling of the cells of the foam material it is preferred that the step of filling the cells of the foam material is carried out a plurality of times, typically twice. More particularly, the step of substantially filling the cells of the foam material with a ceramic slip preferably includes the steps of immersing the foam material in the ceramic slip, drying the ceramic slip and again immersing the foam material in the ceramic slip. We have found that carrying out the impregnation twice greatly facilitates the filling of the cells of the foam material.

We have also found that filling of the cells of the foam material with the ceramic slip is assisted if the ceramic slip is relatively viscous. Preferably the viscosity of the ceramic slip is more than 6000 cps and more preferably more than 8000 cps. Preferably the foam material is placed in a low pressure environment for each immersion of the material in the ceramic slip. Filling of the foam material by the slip is facilitated if most of the air is removed from the foam material.

The porous properties of the bone substitute material formed by the method of the invention are determined to a considerable extent by the properties of the foam material. In certain applications it will be desirable for the bone material to have the same properties throughout its volume and in that ordinary case a foam material having the same structure throughout its volume can be employed. In other applications, however, it may be desirable to vary the properties; for example, it may be desirable for there to be a gradual change in the size and/or shape of the cells of the foam material through at least a region of the foam material. While a gradual reduction in cell size may be obtained by providing a corresponding gradual change in the foam material *per se,* another approach is to distort the polymeric foam material into a state, other than its natural state, to provide a variation in the sizes of the cells in the material. For example, the foam material may be compressed in a region to reduce the cell size in that region of the foam material. It will be appreciated that reducing the size of a cell in the foam material serves to increase the porosity of the bone substitute material, since that is a negative image of the foam material.

Instead of, or in addition to, providing a gradual change, there may be a step change in the size and/or shape of the cells of the foam material in a region of the material. For example, two or more pieces of foam material having different open cell structures may,be secured together, for example by sewing, to provide the foam material. In that case the bone substitute material formed may have a step change in its properties of porosity from one region of the bone material to another.

The foam material may be provided in any of a wide range of sizes. In Examples of the invention described below the foam material is in the form of cubes with sides measuring 25 mm. The invention may, however, employ much smaller or bigger pieces of foam material and the pieces may be in shapes other than cubes. For example, the foam material may comprise pieces that, whether or not they are cubes, have a maximum dimension of less than 12 mm. More particularly, pieces of foam material may be of generally cubic shape having sides of a length less than 10 mm and preferably in the range of 2 to 8 mm. Small pieces of foam of the kind described immediately above may be employed to produce individual granules of material. Those granules may together provide a granular material. The properties of the individual granules may all be the same but they may also vary. For example, there may be relatively large granules mixed with relatively small granules. A variety of sizes of granule promotes close packing but it may also be desirable to supply the granules in a series of ranges of size with relatively little size variation within each range. A purchaser can then select whatever mixture of sizes is appropriate for a particular application. The granules may be formed in irregular shapes by using irregular pieces of foam which may be of varying size and/or of varying shape.

The bone substitute material, whether it is granular or not, may be treated after it has been sintered to alter the shape and/or reduce the size of pieces of the material. The treating of the bone substitute material may comprise a crushing step and/or it may comprise a milling step. In either case the pieces of the material may be of a consistent shape and size or may have a variety of shapes and sizes. A variation in size or shape may be of advantage in some applications but if it is desired to have relatively little variation in the size and shape of relatively small pieces of material, it may be desirable to introduce one or more sieving steps.

The sintered bone substitute material may be composed of any suitable ceramic material. Hydroxyapatite (HA, chemical formula Ca₁₀(PO₄)₆(OH)₂,Ca/P=1.67) is one of the preferred materials, together with tricalcium phosphate (TCP, Ca/P=1.50). In the example of the invention described below the sintered product is a mixture of HA and TCP.

According to another aspect of the invention there is provided a bone substitute material comprising a porous sintered ceramic **characterized in that** the material is a negative image of the structure of an open cell foam with pores in the material corresponding to walls of the foam cells and with substantially solid walls of the pores corresponding to cells of the foam.

It should be noted that while the bone substitute material is, for convenience, defined by reference to the open cell foam this aspect of the invention is directed to the material per se and is not limited to any particular method of making the material. Thus, while it is preferred that the bone substitute material is made by the method defined above, that is not essential.

The pores are preferably relatively large with the majority of the macropore volume comprising pores having an equivalent diameter of more than 100µm. Preferably, the majority of the macropore volume comprises pores having an equivalent diameter in the range 100µm to 500µm. Where reference is made in this specification to macropores, that is to be taken as a reference to a pore having an equivalent diameter greater than 10 µm. Pores of smaller diameter are referred to as micropores.

The overall porosity of the bone substitute material may be relatively low, for example less than 15%. Similarly, the density of the bone substitute material may be relatively high, for example more than 2g/cm³.

In examples of the invention described below the compressive strength of the material is more than 20MPa, and more particularly is in the range of 20 to 80 MPa.

For certain applications it is preferred that there is a gradual change in the size and/or shape of the pores of the material through at least a region of the material, and for other applications or the same applications it may be preferred that there is a step change in the size and/or shape of the pores of the material.

The bone substitute material may be a single piece but it may also be a granular material. Preferably the granules have a maximum dimension of less than 12 mm. The granules of the ceramic material may be of generally cubic shape; in that case, the sides of the cubes are preferably 2 to 8 mm in length. The granular material may comprise a multiplicity of granules of bone substitute material with substantially all of the granules being of substantially the same size or with the granules being of varying sizes. The edges of the granules may be rounded or they may be sharp. In examples of the invention, the compressive modulus of the granular material at a load of 1000 N was more than 60 MPa and, more particularly, in the range of 60 to 100 MPa. The compressive modulus is measured in a standard die plunger test.

By way of example, an embodiment of the invention will now be described with reference to the accompanying drawing, of which:

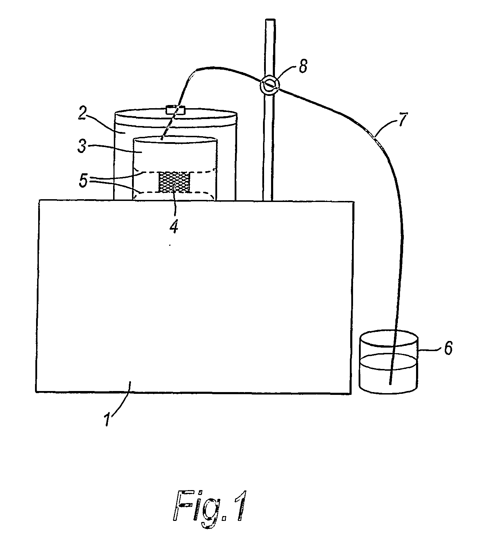

Fig. 1 is a schematic drawing of apparatus for filling a foam material with ceramic slip.

The exemplary method described below employs two starting materials: firstly, an organic foam material having an open cell structure with each cell connecting to each neighbouring cell; secondly, a ceramic slip.

The method is carried out employing the apparatus shown schematically in Fig. 1 where a vacuum pump 1 is shown with a vacuum vessel 2 mounted on its top. Inside the vacuum vessel 2 is a container 3 in the bottom of which a piece of foam material 4 is held in a pair of meshes 5. A beaker 6 contains the ceramic slip and a plastic tube 7 extends from the beaker 6 into the top of the container 3, passing through the lid of the vacuum vessel 2. Partway along the tube 7 an adjuster 8 is provided to control the flow of the slip along the tube 7.

At the start of the method, the adjuster 8 is closed, the foam piece 4 is held in an unstressed state between the meshes 5, which are shaped to provide the desired support, and is placed with the meshes in the bottom of the container 3. The vacuum vessel 2 is then closed and the pressure in the vessel 2 reduced to below atmospheric pressure by operation of the vacuum pump 1.

As the pressure in the vessel 2 reduces, air in the foam piece 4 passes out of the foam, maintaining the pressure inside and outside the foam piece 4 at substantially the same level.

Once the desired low pressure in the vessel 2 has been reached, so that there is substantially no air in the vessel 2, including the foam piece 4, the adjuster 8 is opened a little to allow the ceramic slip in the beaker 6 to be forced up the tube 7 into the container 3. As will be understood, the ceramic slip enters the open cell structure of the foam piece 4 and is unimpeded during that process. Once the foam piece 4 is fully immersed in the ceramic slip the adjuster 8 is closed again. The interior of the vacuum vessel is then vented; during that venting any cells in the foam piece 4 that have not been filled entirely by the ceramic slip are filled.

The foam piece 4 and meshes 5 are then removed from the container 3 and placed on tissue to allow the slip to dry. As the slip dries some slip is observed draining from the foam piece 4.

Once the slip is dry it is replaced, together with the meshes 5, in the container 3 and the steps of evacuation, immersing in the slip, relieving the pressure, removing from the container 3 and drying are all repeated. At the end of these repeated steps, the foam piece 4 is found to be substantially full of ceramic slip material.

The filled foam piece 4 is then slowly heated from room temperature to cause the organic foam to decompose slowly and completely by combustion, the products of the decomposition being allowed to escape from the foam piece 4. The heating is then increased substantially to sinter the ceramic slip and form the bone substitute material.

After sintering, the resulting product can be machined to a desired size and cleaned.

As may be ascertained from the description above, the product generated by the method has ceramic material substantially wherever the foam material had cell spaces, and pores wherever the cell walls of the foam material were present.

Examples of the method of the invention are described below:

### Examples

The Examples employed a piece of polyurethane foam of approximately cubic shape with each side of the cube measuring 25mm. The Examples differed only in the porosities of the foams, which were 20, 30 and 45 ppi.

The ceramic slip material was based on calcium phosphates and used different amounts of powders known as TCP 118 and TCP 130 mixed with distilled water. In the Examples the percentage by weight of each of the components referred to above was:

| | % by weight |
|---|---|
| TCP 118 | 25 |
| TCP 130 | 25 |
| Distilled water | 50 |

The mixture was ball milled in a polyethylene container, using ZrO₂ balls as milling media, for 24 hours.

Then Dispex A40, a non-sodium based dispersant available from Allied Colloids Limited, was added to adjust the viscosity. In the example, the amount of Dispex A40 added was 15ml and this resulted in a viscosity, as measured using a Brookfield viscometer using a spindle speed of 10rpm and a No. 5 spindle, of 10000 cps.

After each immersion of the foam piece in the slip it was dried at room temperature for 24 hours and then dried in an oven at 40°C for another 24 hours.

After completion of the second drying step, the filled foam material was again placed in an oven at a temperature which was increased at the rate of 60°C per hour from room temperature, until a temperature of 600°C was reached. The oven was then held at a temperature of 600°C for 1 hour to complete a first stage of heating. During this slow and relatively gentle heating all of the polyurethane foam decomposed, and the green porous material was left. The temperature of the oven was then further increased for a second stage of heating at the rate of 120°C per hour until it reached 1280°C. The temperature was maintained at 1280°C for 4 hours and then cooled back to room temperature at the rate of 200°C per hour. At the end of the second stage of heating the sintered material had been formed.

The sintered material so formed had an apparent density of approximately 2.6 g/cm³ and an overall porosity of 25% with most of the pores having an equivalent diameter in the range of 100 to 500µm. The material was relatively strong having a compressive strength of between 20 and 80 MPa and there was a high degree of interconnection between adjacent pores with pores generally being in fluid communication with all adjacent pores. Equally, there was a high degree of interconnection of the sintered material with walls around the pores generally being connected to adjacent walls extending in other directions. The sintered material produced comprised biphasic HA and TCP.

The example above was repeated using different solids loadings and different amounts of Dispex A40 to vary the viscosity of the ceramic slip and using different foams. Solids loadings ranging from 40% to 65% by weight of solids were tested, with Dispex A40 added to reduce the viscosity from a maximum of about 18000 cps (with no Dispex A40 added) down to about 5000 cps. The preferred viscosity range was found to be about 6000 to about 10000 cps.

As previously indicated, three foam materials were tried, having porosities of 20 ppi, 30 ppi and 45 ppi. In each case the foam material had a uniform cell structure throughout its volume, with most of the cells having an equivalent diameter in the range of 100 to 250µm. The samples employed were all cubes of the same size as in the example above. Different foam materials resulted in sintered products of different porosities.

A cube of foam was modified by sewing together the opposite faces of the cube at one end to compress that end of the cube to about half its original height. That foam sample resulted in a sintered product whose porosity varied from one end to the other, the porosity being relatively high at the end where the faces were sewn together and relatively low at the opposite end. Such a variation in porosity can be of particular advantage in simulating the bimodal structure of cortical and cancellous bone.

A sample of foam was also prepared by sewing together along adjoining faces a cube of foam of 20 ppi and a cube of foam of 45 ppi. That foam sample resulted in a sintered product whose porosity varied in a step change across a central dividing plane. Despite the discontinuity in the product along that plane, it was found that the mechanical strength of the product was not unduly weakened, presumably because there remain many portions of sintered material traversing the plane.

While in the Examples described above the foam cubes had sides measuring 25mm, the invention may employ much smaller or bigger pieces of foam material. For example, each foam piece may be generally in the shape of a cube whose sides are of a length in the range of 2 to 8 mm. Such small pieces of foam may be employed to produce individual granules of material, which together provide granular material. The precise shapes of the granules are determined by the shapes of the pieces of foam material and any post-treatment of the granules. For example, the granules may be milled after they have been formed to round off their edges. It is also possible to crush larger granules or even larger pieces of material to form granules of a desired size. A range of sizes of granules can be produced in this way. In particular examples, granules in the size range of 2 to 4 mm were prepared and their compressive modulus measured by a standard die plunger test. The modulus at a load of 1000 N was in the range of 60 to 100 MPa.

When the pieces of foam are relatively small it may be advantageous to place many of them together in the vacuum vessel and fill them simultaneously with ceramic slip, rather than treating each piece individually.

## Claims

1. A method of fabricating a bone substitute material, the method comprising the steps of:
providing a foam material (4) having an open cell structure,
introducing a ceramic slip into the cells of the foam material (4),
removing the foam material (4),
sintering the ceramic slip to form a bone substitute material,
**characterized in that**:
when the ceramic slip is introduced into the cells of the foam material (4), the cells of the foam material are substantially filled, and
the bone substitute material formed by the method is substantially a negative image of the foam material (4).

2. A method according to claim 1, in which the step of removing the foam material (4) comprises heating the material.

3. A method according to claim 2, in which the method comprises a first heating step in which the foam material (4) is removed and a second, subsequent, heating step in which the ceramic slip is heated to a higher temperature and is sintered.

4. A method according to any preceding claim, in which the step of substantially filling the cells of the foam material (4) with a ceramic slip includes the steps of immersing the foam material in the ceramic slip, drying the ceramic slip and again immersing the foam material in the ceramic slip.

5. A method according to claim 4, in which the foam material (4) is placed in a low pressure environment for each immersion of the material in the ceramic slip.

6. A method according to any preceding claim, in which there is a gradual change in the size and/or shape of the cells of the foam material (4) through at least a region of the foam material.

7. A method according to any preceding claim, in which the foam material (4) is held in a deformed state during the step of substantially filling the cells of the foam material with ceramic slip.

8. A method according to any preceding claim, in which there is a step change in the size and/or shape of the cells of the foam material (4) in a region of the material.

9. A method according to any preceding claim, in which two or more pieces of foam material (4) having different open cell structures are secured together to provide the foam material.

10. A method according to any preceding claim, in which the foam material (4) comprises pieces having a maximum dimension of less than 12 mm.

11. A method according to any preceding claim, in which the foam material (4) comprises pieces of generally cubic shape having sides of a length less than 10 mm.

12. A method according to any of claims 1 to 10, in which the foam material (4) comprises pieces of irregular shape.

13. A method according to any preceding claim, in which the bone substitute material is a granular material.

14. A method according to any preceding claim, in which the foam material (4) is a polymeric foam material.

15. A bone substitute material comprising a porous sintered ceramic **characterized in that** the material is a negative image of the structure of an open cell foam with pores in the material corresponding to walls of the foam cells and with substantially solid walls of the pores corresponding to cells of the foam.

16. A bone substitute material according to claim 15, in which the overall porosity of the material is less than 15 per cent.

17. A bone substitute material according to claim 15 or 16, in which there is a gradual change in the size and/or shape of the pores of the material through at least a region of the material.

18. A bone substitute material according to any of claims 15 to 17, in which there is a step change in the size and/or shape of the pores of the material.

19. A bone substitute material according to any of claims 15 to 18, in which the density of the material is more than 2g/cm³.

20. A bone substitute material according to any of claims 15 to 19, in which the compressive strength of the material is in the range of 20 to 80 MPa.

21. A bone substitute material according to any of claims 16 to 20, in which the material is a granular material.

22. A bone substitute material according to claim 21 comprising granules having a maximum dimension of less than 12 mm.

23. A bone substitute material according to claim 21 or 22 in which the granules are of irregular shape.

24. A bone substitute material according to any of claims 21 to 23, in which the granular material has a compressive modulus at a load of 1000 N in the range of 60 to 100 MPa.

25. A bone substitute material obtained by a method according to any of claims 1 to 14.

## Patentansprüche

1. Verfahren zur Herstellung eines Knochenersatzmaterials, wobei das Verfahren die Schritte umfasst:
Bereitstellen eines Schaummaterials (4) mit einer offenzelligen Struktur,
Einbringen eines keramischen Gießschlickers in die Zellen des Schaummaterials (4),
Entfernen des Schaummaterials (4),
Sintern des keramischen Gießschlickers, um ein Knochenersatzmaterial zu bilden,
**dadurch gekennzeichnet, dass:**
die Zellen des Schaummaterials im Wesentlichen gefüllt sind, wenn der keramische Gießschlicker in die Zellen des Schaummaterials (4) eingebracht wird, und
das durch das Verfahren gebildete
Knochenersatzmaterial im Wesentlichen ein negatives Abbild des Schaummaterials (4) ist.

2. Verfahren nach Anspruch 1, in welchem der Schritt des Entfernens des Schaummaterials (4) das Erhitzen des Materials umfasst.

3. Verfahren nach Anspruch 2, in welchem das Verfahren einen ersten Erhitzungsschritt umfasst, in welchem das Schaummaterial (4) entfernt wird, und einen zweiten, nachfolgenden Erhitzungsschritt, in welchem der keramische Gießschlicker auf eine höhere Temperatur erhitzt wird und gesintert wird.

4. Verfahren nach irgendeinem vorhergehenden Anspruch, in welchem der Schritt des im Wesentlichen Füllens der Zellen des Schaummaterials (4) mit keramischem Gießschlicker die Schritte enthält: Eintauchen des Schaummaterials in den keramischen Gießschlicker, Trocknen des keramischen Giesschlickers und erneutes Eintauchen des Schaummaterials in den keramischen Gießschlicker.

5. Verfahren nach Anspruch 4, in welchem das Schaummaterial (4) bei jedem Eintauchen des Materials in den keramischen Gießschlicker in eine Niederdruckumgebung gebracht wird.

6. Verfahren nach irgendeinem vorhergehenden Anspruch, in welchem es eine graduelle Änderung in der Größe und/oder Form der Zellen des Schaummaterials (4) durch mindestens einen Bereich des Schaummaterials gibt.

7. Verfahren nach irgendeinem vorhergehenden Anspruch, in welchem das Schaummaterial (4) während des Schrittes des im Wesentlichen Füllens der Zellen des Schaummaterials mit keramischem Gießschlicker in einem deformierten Zustand gehalten wird.

8. Verfahren nach irgendeinem vorhergehenden Anspruch, in welchem es eine sprunghafte Änderung in der Größe und/oder Form der Zellen des Schaumaterials (4) in einem Bereich des Materials gibt.

9. Verfahren nach irgendeinem vorhergehenden Anspruch, in welchem zwei oder mehr Teile des Schaummaterials (4) mit unterschiedlichen offenzelligen Strukturen aneinander befestigt werden, um das Schaummaterial bereitzustellen.

10. Verfahren nach irgendeinem vorhergehenden Anspruch, in welchem das Schaummaterial (4) Teile mit einer maximalen Abmessung von weniger als 12 mm umfasst.

11. Verfahrenen nach irgendeinem vorhergehenden Anspruch, in welchem das Schaummaterial (4) Teile mit im Allgemeinen kubischer Form umfasst, die Seiten mit einer Länge von weniger als 10 mm aufweisen.

12. Verfahren nach irgendeinem der Ansprüche 1-10, in welchem das Schaummaterial (4) Teile mit irregulärer Form umfasst.

13. Verfahren nach irgendeinem vorhergehenden Anspruch, in welchem das Knochenersatzmaterial ein granuläres Material ist.

14. Verfahrenen nach irgendeinem vorhergehenden Anspruch, in welchem das Schaummaterial (4) ein polymeres Schaummaterial ist.

15. Knochenersatzmaterial, umfassend eine poröse gesinterte Keramik, **dadurch gekennzeichnet, dass** das Material ein negatives Abbild der Struktur eines offenzelligen Schaums ist, mit Poren in dem Material, die den Wänden der Zellen des Schaums entsprechen und mit im Wesentlichen festen Wänden der Poren, die den Zellen des Schaums entsprechen.

16. Knochenersatzmaterial nach Anspruch 15, in welchem die Gesamtporosität des Materials weniger als 15 % ist.

17. Knochenersatzmaterial nach Anspruch 15 oder 16, in welchem es eine graduelle Änderung in der Größe und/oder Form der Poren des Materials durch mindestens einen Bereich des Materials gibt.

18. Knochenersatzmaterial nach irgendeinem der Ansprüche 15 bis 17, in welchem es eine sprunghafte Änderung in der Größe und/oder Form der Poren des Materials gibt.

19. Knochenersatzmaterial nach irgendeinem der Ansprüche 15 bis 18, in welchem die Dichte des Materials mehr als 2 g/cm³ beträgt.

20. Knochenersatzmaterial nach irgendeinem der Ansprüche 15 bis 19, in welchem das Material eine Druckbelastbarkeit in dem Bereich von 20 bis 80 MPa aufweist.

21. Knochenersatzmaterial nach irgendeinem der Ansprüche 16 bis 20, in welchem das Material ein granuläres Material ist.

22. Knochenersatzmaterial nach Anspruch 21, umfassend Körnchen mit einer maximalen Abmessung von weniger als 12 mm.

23. Knochenersatzmaterial nach Anspruch 21 oder 22, in welchem die Körnchen eine irreguläre Form aufweisen.

24. Knochenersatzmaterial nach irgendeinem der Ansprüche 21 bis 23, in welchem das granuläre Material ein Kompressionsmodul in dem Bereich von 60 bis 100 MPa bei einer Belastung von 1000 N hat.

25. Knochenersatzmaterial, erhalten durch ein Verfahren nach irgendeinem der Ansprüche 1 bis 14.

## Revendications

1. Procédé de fabrication d'un matériau de substitut osseux, le procédé comprenant les étapes consistant à :
mettre en oeuvre un matériau de mousse (4) ayant une structure à alvéoles ouverts,
introduire une barbotine de céramique dans les alvéoles du matériau de mousse (4),
éliminer le matériau de mousse (4),
fritter la barbotine de céramique pour former un matériau de substitut osseux,
**caractérisé en ce que** :
lorsque la barbotine de céramique est introduite dans les alvéoles du matériau de mousse (4), les alvéoles du matériau de mousse sont sensiblement remplies, et
le matériau de substitut osseux formé par le procédé est sensiblement une image négative du matériau de mousse (4).

2. Procédé selon la revendication 1, dans lequel l'étape d'élimination du matériau de mousse (4) comprend le chauffage du matériau.

3. Procédé selon la revendication 2, dans lequel le procédé comprend une première étape de chauffage, dans laquelle le matériau de mousse (4) est éliminé, et une seconde étape consécutive de chauffage dans laquelle la barbotine de céramique est chauffée à une température supérieure et frittée.

4. Procédé selon l'une quelconque des revendications précédentes, dans lequel l'étape consistant à sensiblement remplir les alvéoles du matériau de mousse (4) avec une barbotine de céramique comprend les étapes consistant à immerger le matériau de mousse dans la barbotine de céramique, à sécher la barbotine de céramique et à immerger à nouveau le matériau de mousse dans la barbotine de céramique.

5. Procédé selon la revendication 4, dans lequel le matériau de mousse (4) est placé dans un environnement sous faible pression pour chaque immersion du matériau dans la barbotine de céramique.

6. Procédé selon l'une quelconque des revendications précédentes, dans lequel il se produit un changement progressif de la taille et/ou de la forme des alvéoles du matériau de mousse (4) à travers au moins une région du matériau de mousse.

7. Procédé selon l'une quelconque des revendications précédentes, dans lequel le matériau de mousse (4) est maintenu dans un état déformé pendant l'étape consistant à remplir sensiblement les alvéoles du matériau de mousse avec une barbotine de céramique.

8. Procédé selon l'une quelconque des revendications précédentes, dans lequel il existe un changement radical de la taille et/ou de la forme des alvéoles du matériau de mousse (4) dans une région du matériau.

9. Procédé selon l'une quelconque des revendications précédentes, dans lequel deux pièces de matériau de mousse (4) ou plus, ayant différentes structures à alvéoles ouvertes, sont fixées ensemble pour fournir le matériau de mousse.

10. Procédé selon l'une quelconque des revendications précédentes, dans lequel le matériau de mousse (4) comprend des pièces ayant une dimension maximale inférieure à 12 mm.

11. Procédé selon l'une quelconque des revendications précédentes, dans lequel le matériau de mousse (4) comprend des pièces présentant une forme globalement cubique ayant des côtés d'une longueur inférieure à 10 mm.

12. Procédé selon l'une quelconque des revendications 1 à 10, dans lequel le matériau de mousse (4) comprend des pièces de forme irrégulière.

13. Procédé selon l'une quelconque des revendications précédentes, dans lequel le matériau de substitut osseux est un matériau granulaire.

14. Procédé selon l'une quelconque des revendications précédentes, dans lequel le matériau de mousse (4) est un matériau de mousse polymère.

15. Matériau de substitut osseux comprenant une céramique frittée poreuse, **caractérisé en ce que** le matériau est une image négative de la structure d'une mousse à alvéoles ouvertes avec des pores dans le matériau correspondant aux parois des alvéoles de mousse et avec des parois sensiblement continues des pores correspondants aux alvéoles de la mousse.

16. Matériau de substitut osseux selon la revendication 15, dans lequel la porosité globale du matériau est inférieure à 15 pourcent.

17. Matériau de substitut osseux selon la revendication 15 ou 16, dans lequel il se produit un changement progressif de la taille et/ou de la forme des pores du matériau à travers au moins une région du matériau.

18. Matériau de substitut osseux selon l'une quelconque des revendications 15 à 17, dans lequel il se produit un changement radical dans la taille et/ou dans la forme des pores du matériau.

19. Matériau de substitut osseux selon l'une quelconque des revendications 15 à 18, dans lequel la masse volumique du matériau est supérieure à 2 g/cm³.

20. Matériau de substitut osseux selon l'une quelconque des revendications 15 à 19, dans lequel la résistance à la compression du matériau se situe dans la plage de 20 à 80 MPa.

21. Matériau de substitut osseux selon l'une quelconque des revendications 16 à 20, dans lequel le matériau est un matériau granulaire.

22. Matériau de substitut osseux selon la revendication 21, comprenant des granulés ayant une dimension maximale inférieure à 12 mm.

23. Matériau de substitut osseux selon la revendication 21 ou 22, dans lequel les granulés présentent une forme irrégulière.

24. Matériau de substitut osseux selon l'une quelconque des revendications 21 à 23, dans lequel le matériau granulaire présente un module de compression à une charge de 1000 N qui se situe dans la plage de 60 à 100 MPa.

25. Matériau de substitut osseux obtenu par un procédé selon l'une quelconque des revendications 1 à 14.
